# EUROPEAN PATENT APPLICATION

(11) **EP 4 474 373 A1**
(43) Date of publication of application: **11.12.2024**
(21) Application number: 24741602.7
(22) Date of filing: 12.01.2024
(51) Int. Cl.: C07C 17/23, C07C 17/00, C07C 17/25, C07C 19/08, C07C 21/18, B01J 23/42, B01J 23/44, B01J 23/46, C07B 61/00

(54) **FLUOROETHANE COMPOUND PRODUCTION METHOD AND FLUOROOLEFIN PRODUCTION METHOD**

(30) Priority: 13.01.2023 JP 2023003900; 06.04.2023 JP 2023062394
(71) Applicant: DAIKIN INDUSTRIES, LTD., Osaka-shi, Osaka 530-0001 (JP)
(72) Inventor: TAKAHASHI, Kazuhiro, Osaka-Shi, Osaka 530-0001 (JP); SHIMADA, Tomoo, Osaka-Shi, Osaka 530-0001 (JP); HIRANO, Akihisa, Osaka-Shi, Osaka 530-0001 (JP); YOSHIMURA, Toshikazu, Osaka-Shi, Osaka 530-0001 (JP); KOMATSU, Yuzo, Osaka-Shi, Osaka 530-0001 (JP); CHAKI, Takehiro, Osaka-Shi, Osaka 530-0001 (JP)
(74) Representative: Hoffmann Eitle
(86) International application number: PCT/JP2024/000706
(87) International publication number: WO 2024/150830

(57) **Abstract**

Provided is a method for producing a fluoroethane compound, the method being capable of efficiently producing a fluoroethane compound, which is a desired product. The method comprises step A of obtaining a product comprising a fluoroethane compound by performing a reduction reaction of the chlorofluoroethane compound of the present disclosure in the presence of a catalyst, wherein the catalyst is a catalyst in which at least one metal selected from the group consisting of Ni, Pd, Pt, Ru, and Rh is supported on activated carbon. The production method according to the present disclosure enables efficient production of the desired fluoroethane compound.

## Description

### Technical Field

The present disclosure relates to a method for producing a fluoroethane compound and a method for producing a fluoroolefin.

### Background Art

Fluoroethanes typified by 1,1,2-trifluoroethane (hereinafter sometimes referred to as "HFC-143") are known as a starting material for producing various refrigerants. Various methods have been proposed for the production of fluoroethanes such as HFC-143.

For example, Patent Literature 1 proposes a technique for producing HFC-143 by a hydrogenation reaction of chlorotrifluoroethylene or the like, in the presence of a hydrogenation catalyst. Patent Literature 2 discloses a process of obtaining HFO-1123 by a reaction of chlorotrifluoroethylene (CTFE) and hydrogen, in which HFC-143 and HCFC-133b are included as by-products.

### Citation List

### Patent Literature

PTL 1: JPH01-287044A
PTL 2: JP2016-130236A

### Summary of Invention

### Technical Problem

An object of the present disclosure is to efficiently produce a fluoroethane compound, which is a desired product.

The present invention has been accomplished in view of the above. An object of the present disclosure is to provide a method for producing a fluoroethane compound, the method being capable of efficiently producing a fluoroethane compound, which is a desired product.

### Solution to Problem

For example, the present disclosure encompasses the subject matter described in the following items.

### Item 1.

A method for producing a fluoroethane compound, comprising
step A of obtaining a product comprising a fluoroethane compound by performing a reduction reaction of a chlorofluoroethane compound in the presence of a catalyst and hydrogen,
wherein the catalyst is a catalyst in which at least one metal selected from the group consisting of Ni, Pd, Pt, Ru, and Rh is supported on activated carbon.

### Item 2.

The production method according to Item 1,
wherein the fluoroethane compound comprises 1,1,2-trifluoroethane, and the chlorofluoroethane compound comprises 1,1,2-trichloro-1,2,2-trifluoroethane.

### Item 3.

The production method according to Item 1,
wherein the fluoroethane compound comprises 1,1,2-trifluoroethane, and the chlorofluoroethane compound comprises 1-chloro-1,1,2-trifluoroethane.

### Item 4.

The production method according to Item 1,
wherein the fluoroethane compound comprises 1,1-difluoroethane, and the chlorofluoroethane compound comprises 1-chloro-1,1,2-trifluoroethane.

### Item 5.

The production method according to any one of claims 1 to 4, wherein the product comprises at least one compound selected from the group consisting of chlorotrifluoroethylene and trifluoroethylene as a by-product.

### Item 6.

The production method according to Item 2,
comprising
obtaining the 1,1,2-trichloro-1,2,2-trifluoroethane by a liquid-phase fluorination reaction of tetrachloroethylene.

### Item 7.

A method for producing a fluoroolefin, comprising
obtaining a fluoroethane compound by the production method of any one of Items 1 to 6, and
obtaining the fluoroolefin by a dehydrofluorination reaction of the fluoroethane compound obtained in the above step.

### Advantageous Effects of Invention

The production method according to the present disclosure enables efficient production of the desired fluoroethane compound.

### Brief Description of Drawings

Fig. 1 is a schematic diagram of a comprehensive process including a method for producing a fluoroethane compound according to the present disclosure.
Fig. 2 is a schematic diagram of another embodiment of a comprehensive process including a method for producing a fluoroethane compound according to the present disclosure.

### Description of Embodiments

For producing a fluoroethane compound through a hydrogenation reaction of chlorotrifluoroethylene, as stated above, since chlorotrifluoroethylene is expensive, there is currently a demand for producing a fluoroethane compound by using a starting material at even lower cost and also in an efficient manner.

The inventors conducted extensive research to achieve the object of producing the desired fluoroethane compound at high conversion and high selectivity. The inventors consequently found that the above object can be achieved by performing a gas-phase reduction reaction of a chlorofluoroethane compound using hydrogen in the presence of a catalyst.

Embodiments included in the present disclosure are described in detail below. In the present specification, the terms "comprise" and "contain" include the concepts of "comprise," "contain," "consist essentially of," and "consist of."

Numerical ranges expressed by using the term "to" in the present specification indicate ranges that include numerical values shown before and after the "to" as the minimum and maximum values, respectively. In numerical ranges described stepwise in the present specification, the upper-limit value or the lower-limit value of one numerical range can be randomly combined with the upper-limit value or the lower-limit value of another numerical range. In the present specification, the upper-limit values or the lower-limit values of the numerical ranges stated in the present specification may be replaced with a value shown in the Examples or a value that can be unambiguously derived from the Examples.

### 1. Production Method for Fluoroethane Compound

The method for producing a fluoroethane compound according to the present disclosure comprises the following step A:
Step A: obtaining a product comprising a fluoroethane compound by performing a reduction reaction of a chlorofluoroethane compound in the presence of a catalyst.

In the method for producing a fluoroethane compound according to the present disclosure, the catalyst is a catalyst in which at least one metal selected from the group consisting of Ni, Pd, Pt, Ru, and Rh is supported on activated carbon.

The production method according to the present disclosure enables efficient production of the desired fluoroethane compound. That is, the production method according to the present disclosure achieves a high conversion and also a high selectivity of the desired fluoroethane compound.

Hereinafter, in the present specification, the method for producing a fluoroethane compound according to the present disclosure is referred to as "Production Method 1 according to the present disclosure."

In Production Method 1 according to the present disclosure, in step A, a reduction reaction of a starting material containing a chlorofluoroethane compound is performed in the presence of a catalyst and hydrogen. By the reduction reaction, a product comprising the desired fluoroethane compound is obtained.

### Starting Material

The starting material for use in step A contains a chlorofluoroethane compound. The chlorofluoroethane compound is a compound that contains two carbon atoms and contains both chloro and fluoro groups. The chlorofluoroethane compound preferably contains two or more, more preferably three or more, and even more preferably three fluoro groups.

Specific examples of the chlorofluoroethane compound include at least one member selected from the group consisting of 1,1,2-trichloro-1,2,2-trifluoroethane (CFC-113), 1-chloro-1,1,2-trifluoroethane (HCFC-133b), 1-chloro-1-fluoroethane (HCFC-151), 1,2-dichloro-1,2-difluoroethane (HCFC-132), 2-chloro-1,1-difluoroethane (HCFC-142), 1-chloro-1,2-difluoroethane (HCFC-142a), 2-chloro-1,1,2-trifluoroethane (HCFC-133), 2,2-dichloro-1,1,1-trifluoroethane (HCFC-123), and 1,2-dichloro-1,2,2-trifluoroethane (HCFC-123a).

In particular, the chlorofluoroethane compound more preferably comprises at least one member selected from the group consisting of 1,1,2-trichloro-1,2,2-trifluoroethane (CFC-113) and 1-chloro-1,1,2-trifluoroethane (HCFC-133b), and particularly preferably comprises 1,1,2-trichloro-1,2,2-trifluoroethane (CFC-113). That is, the starting material for use in step A particularly preferably contains 1,1,2-trichloro-1,2,2-trifluoroethane (CFC-113). In this case, a fluoroethane compound can be efficiently obtained, and the conversion can be further improved.

The chlorofluoroethane compound for use in step A preferably comprises 50% by mass or more, more preferably 80% by mass or more, even more preferably 90% by mass or more, and particularly preferably 95% by mass or more of CFC-113. CFC-113 alone may also be used as the chlorofluoroethane compound for use in step A.

The chlorofluoroethane compound for use in step A preferably comprises 50% by mass or more, more preferably 80% by mass or more, even more preferably 90% by mass or more, and particularly preferably 95% by mass or more of HCFC-133b. HCFC-133b alone may also be used as the chlorofluoroethane compound for use in step A.

The starting material for use in step A may consist of the chlorofluoroethane compound, or may contain other components in addition to the chlorofluoroethane compound as long as the effects of the present disclosure are not impaired.

The water content in the chlorofluoroethane compound for use in step A is preferably 100 ppm by mass or less, more preferably 70 ppm by mass or less, even more preferably 60 ppm by mass or less, and particularly preferably 50 ppm by mass or less, based on the total mass of the chlorofluoroethane compound. In this case, catalyst degradation is suppressed, making it possible to improve the catalyst life. The lower limit of the water content in the chlorofluoroethane compounds is not limited as long as the effects of Production Method 1 according to the present disclosure are not impaired. For example, in Production Method 1 according to the present disclosure, the chlorofluoroethane compound introduced into the reactor may not contain water. Alternatively, the lower limit of the water content in the chlorofluoroethane compound introduced into the reactor can be 0.1 ppm by mass. From the perspective of reducing the complexity of the process for removing water from the chlorofluoroethane compound introduced into the reactor, the lower limit of the water content in the chlorofluoroethane compound introduced into the reactor is preferably set to 1 ppm by mass. The water content in the chlorofluoroethane used in Production Method 1 according to the present disclosure is the value measured with a Karl Fischer moisture measurement system.

### Catalyst

In Production Method 1 according to the present disclosure, in step A, a catalyst is used. Specifically, in step A, a catalyst in which at least one metal selected from the group consisting of Ni, Pd, Pt, Ru, and Rh is supported on activated carbon is used as stated above. In particular, from the viewpoint of more efficiently obtaining a fluoroethane compound, the catalyst for use is preferably a catalyst in which at least one metal selected from the group consisting of Pd, Pt, and Ru is supported on activated carbon, more preferably a catalyst in which at least one metal selected from the group consisting of Pd and Pt is supported on activated carbon, and even more preferably a catalyst in which Pd is supported on activated carbon.

In the catalyst, the amount of the supported metal is not limited. The content of the metal based on the total amount of the carrier (activated carbon) is preferably 0.1% by mass or more, more preferably 0.2% by mass or more, and particularly preferably 0.5% by mass or more, from the viewpoint of efficiently obtaining a fluoroethane compound. Moreover, from the viewpoint of more efficiently obtaining a fluoroethane compound more, the content of the metal based on the total amount of the carrier (activated carbon) is preferably 10% by mass or less, more preferably 5% by mass or less, and particularly preferably 3% by mass or less.

The preparation method for the catalyst is not limited and a wide range of known preparation methods can be used. For example, the catalyst can be obtained by a method comprising immersing activated carbon, which is a carrier, in a solution containing at least one metal selected from the group consisting of Ni, Pd, Pt, Ru, and Rh to impregnate the carrier with the solution, followed by, if necessary, neutralization, calcination, and the like. In this case, the amount of noble metal supported on the carrier can be controlled by adjusting the concentration of the solution, the impregnation time, and the like.

The type of activated carbon is not limited. For example, a wide variety of commercially available activated carbons can be used. Mainly, activated carbons, such as woodbased activated carbon, coal-based activated carbon, coconut shell activated carbon, oil carbon activated carbon, and phenolic resin activated carbon, can be suitably used. Of these, woodbased activated carbon, coal-based activated carbon, and coconut shell activated carbon are more suitably used.

In step A, the amount of the catalyst for use is not limited, and the amount can be, for example, the same as or similar to the amount used in known hydrogenation reactions. For example, the amount of the catalyst for use can be appropriately set according to the size of the reactor used in the reduction reaction of step A, the amount of the starting material used, and the like.

### Reduction Reaction

In step A, a gas-phase reduction reaction of the starting material containing the chlorofluoroethane compound is performed using hydrogen in the presence of a catalyst. One embodiment of the reduction reaction is a dehydrohalogenation reaction, in which a halogen element in a molecule is substituted with hydrogen, and hydrogen halide is released. Another embodiment of the reduction reaction is a hydrogen addition reaction, in which a hydrogen molecule is added to a double bond in a molecule to form an alkane from an alkene. In step A, either or both of the above reactions proceed.

In the reduction reaction of step A, the amount of hydrogen for use is not limited, and can be, for example, 3 mol or more and 60 mol or less, per mole of the chlorofluoroethane compound in the starting material. It is preferable to supply to the reactor hydrogen in an amount equal to or more than the equivalent of the starting material, together with the starting material, whereby effects of improving the selectivity of HFC-143 and suppressing catalyst degradation can be expected to be achieved. The amount of hydrogen is preferably 5 mol or more, and more preferably 7 mol or more, per mole of the chlorofluoroethane compound in the starting material. By using an appropriate amount of hydrogen, effects of improving the selectivity of HFC-143 and suppressing catalyst degradation can be expected to be achieved. On the other hand, from the perspective of efficiently operating the plant, it is necessary to keep the excess amount of hydrogen within a moderate range because excessive supply of hydrogen can lead to losses of hydrogen and HFC-143. Therefore, the amount of hydrogen is preferably 55 mol or less, more preferably 40 mol or less, even more preferably 25 mol or less, and particularly preferably 21 mol or less, per mole of the chlorofluoroethane compound in the starting material. As stated above, the chlorofluoroethane compound for use in step A may have a water content, and when the water content is 100 ppm by mass or less based on the total mass of the chlorofluoroethane compound, catalyst degradation is likely to be suppressed. In view of this, the amount of hydrogen is preferably 15 mol or more, and more preferably 20 mol or more, and is preferably 60 mol or less, more preferably 55 mol or less, and even more preferably 40 mol or less, per mole of the chlorofluoroethane compound in the starting material.

The method for performing the reduction reaction is not limited. For example, a gas-phase reduction reaction is performed in a reactor containing a catalyst by introducing the starting material containing the chlorofluoroethane compound and hydrogen gas into the reactor and bringing them into contact with the catalyst. Subsequently, a product comprising the desired fluoroethane compound is collected outside the reactor, for example, as a mixed gas. The mixed gas can contain not only the desired fluoroethane compound, but also the by-products described below, unreacted starting materials, and the like. In the reduction reaction, the starting material containing the chlorofluoroethane compound and hydrogen gas may be introduced into the reactor from the same line (pipe) or from different lines (pipes).

In the reduction reaction, the reaction temperature (the ambient temperature when the starting material is brought into contact with the catalyst) is not limited and may be, for example, 50 to 400°C, preferably 100 to 380°C, more preferably 100 to 350°C, and particularly preferably 150 to 330°C.

The reaction time of the reduction reaction is not limited. For example, the contact time represented by W/F, i.e., the ratio of the catalyst amount in the reactor W (g) to the total flow rate of the chlorofluoroethane compound and hydrogen gas introduced into the reactor F, may be 1 to 50 g·sec/cc, preferably 5 g·sec/cc or more, and more preferably 10 g·sec/cc or more, and is preferably 40 g·sec/cc or less, more preferably 30 g·sec/cc or more, and even more preferably 20 g·sec/cc or less.

The reduction reaction of step A may be performed either continuously or batch-wise. Further, the (gas-phase) reduction reaction of step A can be performed under reduced pressure, atmospheric pressure, or increased pressure. For example, from the perspective of reactivity, the pressure during the reaction is preferably 2 MPaG or less, more preferably 1.5 MPaG or less, and particularly preferably 1 MPaG or less. The G in "MPaG" means gauge pressure and indicates the value displayed on a pressure gauge relative to atmospheric pressure (i.e., atmospheric pressure = 0 MPaG). The reduction reaction can also be performed by allowing an inert gas, such as helium, nitrogen, or argon, to be present together with the reaction gas.

The reactor for use in the reduction reaction may be, for example, a tubular flow reactor or the like. For example, the flow reactor may be an adiabatic reactor or a multitubular reactor in which the temperature can be controlled by heating with a heating medium while controlling the reaction temperature by removing reaction heat. The reactor is preferably formed of a material that is resistant to corrosive action, and is particularly preferably formed of stainless steel (SUS), Hastelloy, Inconel, Monel, or the like.

The reactor may also be provided with a jacket for adjusting the temperature inside the reactor. For example, a heating medium or the like may be circulated in the jacket. This makes it possible to adjust the temperature of the gases (e.g., the chlorofluoroethane compound as a starting material, and hydrogen, as well as hydrogen chloride, fluoroethanes, and fluoroethylenes as reaction products) in the reactor.

The desired fluoroethane compound can be obtained by collecting the mixed gas that is obtained by performing the reduction reaction of step A described above. In addition to step A, Production Method 1 according to the present disclosure may also comprise other steps, as necessary. The gas at the outlet of the reactor contains, for example, chlorofluoroethane as an unreacted starting material, and hydrogen, as well as hydrogen chloride, fluoroethanes, and fluoroethylenes as reaction products. Therefore, Production Method 1 according to the present disclosure can comprise steps to facilitate separation of these gases, such as compression for increasing the pressure of the gases, cooling for making separation easy, distillation, deacidification, and dehydration.

### Product

The product of Production Method 1 according to the present disclosure comprises the fluoroethane compound. In the present specification, the fluoroethane compound is a saturated hydrocarbon containing two carbon atoms and one or more fluorine atoms. The type of the fluoroethane compound is not limited as long as it corresponds to the type of the chlorofluoroethane compound for use in step A. The fluoroethane compound can contain a chloro group. That is, the fluoroethane compound may be a chlorofluoroethane compound; however, the fluoroethane compound preferably does not contain a chloro group.

In Production Method 1 according to the present disclosure, one or more fluoroethane compounds described above are produced. That is, in Production Method 1 according to the present disclosure, the product comprises one or more fluoroethane compounds described above.

Specific examples of chlorofluoroethane compounds and/or fluoroethane compounds obtained by the Production Method 1 according to the present disclosure include 1,1,2-trifluoroethane (HFC-143), fluoroethane (HFC-161), 1,2-difluoroethane (HFC-152), 1,1-difluoroethane (HFC-152a), 2-chloro-1,1,2-trifluoroethane (HCFC-133), 1-chloro-1,1,2-trifluoroethane (HCFC-133b), 2,2-dichloro-1,1,1-trifluoroethane (HCFC-123), 1,2-dichloro-1,2,2-trifluoroethane (HCFC-123a), 1,1,1-trifluoroethane (HFC-143a), 1,1,2-trifluoroethane (HFC-143), 1,1,2,2-tetrafluoroethane (HFC-134), 1,1,1,2-tetrafluoroethane (HFC-134a), and the like.

Just to note, in Production Method 1, the starting material used in step A and the product are different. For example, when the starting material for use in step A is HCFC-133b, the product is a compound other than HCFC-133b.

The fluoroethane compound obtained in Production Method 1 according to the present disclosure preferably comprises 1,1,2-trifluoroethane (HFC-143). In particular, the fluoroethane compound obtained in Production Method 1 according to the present disclosure preferably comprises HFC-143 as a main product. This is because when 1,1,2-trifluoroethane is the main product, a particularly high conversion and a particularly high selectivity are achieved. When HFC-143 is the main product, the chlorofluoroethane compound as a starting material is 1,1,2-trichloro-1,2,2-trifluoroethane (CFC-113) or 1-chloro-1,1,2-trifluoroethane (HCFC-133b).

The term "main product" as used herein means a component that is present in an amount of 50 mol% or more in the product.

When HFC-143 is the main product, the content of HFC-143 in the product is preferably 60 mol% or more, and more preferably 70 mol% or more.

The fluoroethane compound obtained in Production Method 1 according to the present disclosure may be 1,1-difluoroethane (HFC-152a). That is, the fluoroethane compound obtained in Production Method 1 according to the present disclosure may comprise HFC-152a as the main product. When HFC-152a is the main product, the chlorofluoroethane compound as a starting material is 1-chloro-1,1,2-trifluoroethane (HCFC-133b).

The product obtained in Production Method 1 according to the present disclosure may comprise by-products, unreacted starting materials, and the like. The content of the by-products in the product obtained in Production Method 1 according to the present disclosure can be, for example, 40 mol% or less, more preferably 30 mol% or less, and even more preferably 20 mol% or less, based on the total amount of the product.

The type of the by-products is not limited. For example, the by-products may comprise at least one compound selected from the group consisting of chlorotrifluoroethylene and trifluoroethylene. That is, the product obtained in step A can comprise at least one compound selected from the group consisting of chlorotrifluoroethylene and trifluoroethylene as a by-product.

For example, when CFC-113 is used as the chlorofluoroethane compound, i.e., the starting material, the main product will be HFC-143, and at least one compound selected from the group consisting of chlorotrifluoroethylene and trifluoroethylene will likely be formed as a by-product. Chlorotrifluoroethylene and trifluoroethylene are intermediates, for example, in a reduction reaction for obtaining HFC-143 from CFC-113.

More specifically, when the main component in the product obtained in Production Method 1 according to the present disclosure is HFC-143, 1,2-dichloro-1,2,2-trifluoroethane (HCFC-123a) can be produced as a by-product. When HFC-143 is the main product, the content of the by-product in the product is preferably less than 40 mol%, more preferably less than 30 mol%, and even more preferably less than 20 mol%.

Further, when the main component in the product obtained in Production Method 1 according to the present disclosure is HFC-152a, 1,2-dichloro-1,2,2-trifluoroethane (HCFC-123a) can be produced as a by-product. When HFC-152a is the main product, the content of the by-product in the product is preferably less than 40 mol%, more preferably less than 30 mol%, and even more preferably less than 20 mol%.

When the by-product comprises at least one compound selected from the group consisting of chlorotrifluoroethylene and trifluoroethylene, the total content of chlorotrifluoroethylene and trifluoroethylene can be less than 40 mol%, more preferably less than 30 mol%, and even more preferably less than 20 mol%, based on the total amount of the product.

The product obtained in Production Method 1 according to the present disclosure may be purified to increase the purity of the desired compound, or the product obtained without purification may be used as the desired compound. Moreover, when the product contains an unreacted starting material, the starting material can be separated by an appropriate method and used again as a starting material for the reaction. Specifically, in Production Method 1, the crude product can be recycled for use as a starting material.

In Production Method 1 according to the present disclosure, for example, a composition comprising HFC-143 and HCFC-123a can be obtained. In this composition, the content of HFC-143 is preferably 60 mol% or more, and more preferably 70 mol% or more, based on the total amount of HFC-143 and HCFC-123a. The content of HCFC-123a can be less than 40 mol%, preferably less than 30 mol%, and even more preferably less than 20 mol%, based on the total amount of HFC-143 and HCFC-123a.

Further, in Production Method 1 according to the present disclosure, for example, a composition comprising HFC-152a and HCFC-123a can be obtained. In this composition, the content of HFC-152a is preferably 60 mol% or more, and more preferably 70 mol% or more, based on the total amount of HFC-152a and HCFC-123a. The content of HCFC-123a can be less than 40 mol%, preferably less than 30 mol%, and more preferably less than 20 mol%, based on the total amount of HFC-152a and HCFC-123a.

### Production Method for Starting Material

The method for producing the chlorofluoroethane compound for use as the starting material in step A is not limited and, for example, a wide range of known production methods can be used. In particular, the chlorofluoroethane compound is preferably produced by a method that comprises step A0 of reacting tetrachloroethylene with a fluorinating agent. In this case, for example, 1,1,2-trichloro-1,2,2-trifluoroethane (CFC-113) is likely to be produced as the chlorofluoroethane compound.

In step A0, the reaction between tetrachloroethylene and a fluorinating agent is, for example, a liquid-phase fluorination reaction. That is, Production Method 1 according to the present disclosure may also comprise obtaining the 1,1,2-trichloro-1,2,2-trifluoroethane by a liquid-phase fluorination reaction of tetrachloroethylene.

In step A0, the fluorinating agent for use is not limited and may be, for example, a fluorinating agent (fluorination catalyst), such as HF or a metal halide. Examples of the fluorinating agent (fluorination catalyst) include SbCl₅, SbCl₃, SnCl₄, TaCl₅, TiCl₄, NbCl₅, MoCl₆, and FeCl₃, and those in which some or all of the Cl bonded to these fluorinating agents are substituted with fluorine, mixtures thereof, and combinations thereof. Among these, antimony pentachloride and one in which some of the Cl in antimony pentachloride are substituted with fluorine are preferable, and a liquid-phase fluorination reaction in which HF is used in combination is suitably used in the reaction. In the case of a liquid-phase fluorination reaction as an example, this reaction is performed at a reaction temperature of 50 to 200°C and a reaction pressure of 0.2 to 1.5 MPaG. To maintain the valence of antimony, which is a fluorinating agent, as 5, the addition of chlorine may also be performed as appropriate. The reaction gas that flows out of the reactor can contain not only the desired CFC-113, but also unreacted HF, HCl, CFC-112 as an intermediate, chlorine, and the like. In the reaction performed in step A0, commonly used reactors formed of corrosion-resistant materials etc. can be used. For example, materials containing nickel, such as Hastelloy, Inconel, and Monel, are suitably used.

As described above, Production Method 1 according to the present disclosure can comprise step A0 before step A.

### 2. Production Method for Fluoroolefin

A fluoroolefin can also be obtained by a dehydrohalogenation reaction of the fluoroethane compound obtained in Production Method 1 according to the present disclosure. That is, the present disclosure also encompasses a method for producing a fluoroolefin through the step of dehydrofluorination of the fluoroethane compound obtained in Production Method 1 according to the present disclosure. Hereinafter, this method is referred to as "Production Method 2 according to the present disclosure." Further, the dehydrofluorination step performed in Production Method 2 according to the present disclosure is referred to as "step B."

In Production Method 2 according to the present disclosure, for example, a fluoroolefin represented by the following formula (4) can be obtained:

CX¹¹X²¹=CX³¹X⁴¹ (4)

wherein X¹¹, X²¹, X³¹, and X⁴¹ are the same or different and each represents a hydrogen atom, a fluorine atom, or a chlorine atom; at least one of X¹¹, X²¹, X³¹, and X⁴¹ represents a hydrogen atom; and at least one of X¹¹, X²¹, X³¹, and X⁴¹ represents a fluorine atom.

In the dehydrofluorination step, the method for the dehydrofluorination reaction is not limited. For example, the dehydrofluorination reaction may be performed under conditions that are the same as or similar to those of known dehydrofluorination reactions. For example, the dehydrofluorination reaction may be performed in a gas phase, in the presence of a catalyst for dehydrofluorination.

In Production Method 2 according to the present disclosure, the dehydrofluorination reaction when 1,1,2-trifluoroethane (HFC-143) is used as a fluoroethane is performed according to the following reaction scheme.

CF₂HCFH₂ → CHF=CHF + HF

The catalyst for dehydrofluorination is not limited, and a wide range of known catalysts can be used. Examples include chromium oxide, fluorinated chromium oxide, aluminum oxide, fluorinated aluminum oxide, and the like.

The catalyst for dehydrofluorination may be supported on a carrier. Examples of carriers include carbon, alumina (Al₂O₃), zirconia (ZrO₂), silica (SiO₂), titania (TiO₂), and the like. As carbon, activated carbon, amorphous carbon, graphite, diamond, or the like can be used.

In Production Method 2 according to the present disclosure, the dehydrofluorination reaction may also be performed in the presence of an oxidizing agent. In the dehydrofluorination reaction, the oxidizing agent is preferably supplied together with the reaction gas from the viewpoint that the effect of suppressing catalyst degradation is likely to improve. Examples of oxidizing agents include oxygen, chlorine, bromine, iodine, and the like. Oxygen is particularly preferable. The concentration of the oxidizing agent is not limited; and may be, for example, the same as or similar to that in known dehydrofluorination reactions.

The reaction temperature in the dehydrofluorination reaction is also not limited; and may be the same as or similar to that in known dehydrofluorination reactions. For example, the reaction temperature in the dehydrofluorination reaction may be, for example, 300°C or more, preferably 320°C or more, more preferably 340°C or more, and particularly preferably 350°C or more. The reaction temperature in the dehydrofluorination reaction may also be 600°C or less, preferably 550°C or less, more preferably 500°C or less, and particularly preferably 450°C or less.

The reaction time of the dehydrofluorination reaction and the pressure during the reaction are also not limited, and a wide range of known conditions can be adopted. The dehydrofluorination reaction may also be performed either in the presence of an inert gas, or in the presence of air or oxygen. The dehydrofluorination reaction may be performed either continuously or batch-wise.

Production Method 2 according to the present disclosure may also comprise, if necessary, other steps in addition to the dehydrofluorination step. Also in Production Method 2, the starting material can be separated from the crude product obtained in Production Method 2, and recycled.

The desired fluoroolefin of Production Method 2 according to the present disclosure, for example, a compound represented by formula (4), is obtained by the dehydrofluorination step. In the dehydrofluorination step, one or more fluoroolefins are obtained.

The resulting fluoroolefin can depend on the fluoroethane used in the dehydrofluorination step. Examples of fluoroolefins include 1,2-difluoroethylene (HFO-1132), 1,1-difluoroethylene (HFO-1132a), trifluoroethylene (HFO-1123), fluoroethylene (HFO-1141), and the like.

In Production Method 2 according to the present disclosure, when HFC-143 is used as a fluoroethane, the resulting fluoroolefin is HFO-1132. In Production Method 2 according to the present disclosure, when HFC-143a is used as a fluoroethane, the resulting fluoroolefin is HFO-1132a. In Production Method 2 according to the present disclosure, when HFC-134a is used as a fluoroethane, the resulting fluoroolefin is HFO-1123. HFO-1132 can include trans-1,2-difluoroethylene [(E)-HFO-1132] and *cis-*1,2-difluoroethylene [(Z)-HFO-1132].

To obtain a fluoroolefin by Production Method 2 according to the present disclosure, Production Method 1 according to the present disclosure and Production Method 2 according to the present disclosure may be performed consecutively or may be performed independently. In Production Method 2 according to the present disclosure, after a product comprising a fluoroethane compound is obtained through step A described above, a fluoroolefin can be produced through the dehydrofluorination step of the fluoroethane compound.

As described above, Production Method 2 according to the present disclosure is a method for producing a fluoroolefin, comprising obtaining a fluoroethane compound by Production Method 1 according to the present disclosure and obtaining the fluoroolefin by a dehydrofluorination reaction of the fluoroethane compound obtained in the above step.

### 3. Comprehensive Process

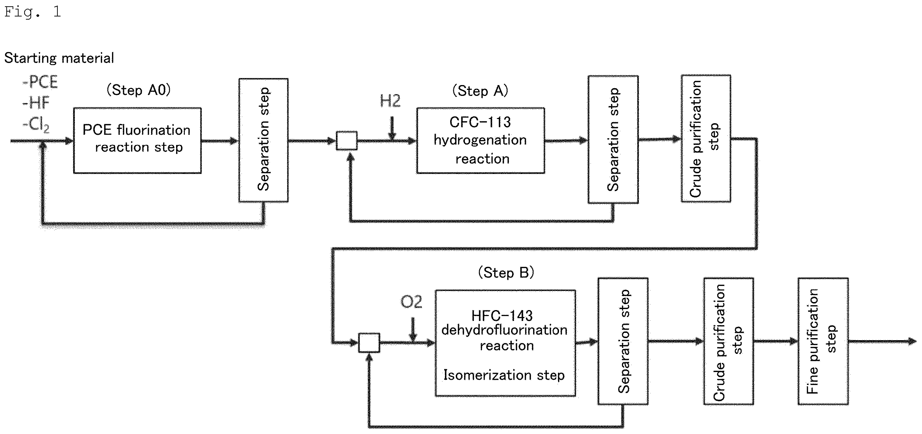

The present disclosure encompasses a comprehensive process comprising step A0, step A, and step B described above. This comprehensive process is referred to as "Comprehensive Process 1." Fig. 1 shows the concept of Comprehensive Process 1. In Fig. 1, PCE means perchloroethylene (tetrachloroethylene). This figure is a conceptual diagram of Comprehensive Process 1 and does not show all flows or processes.

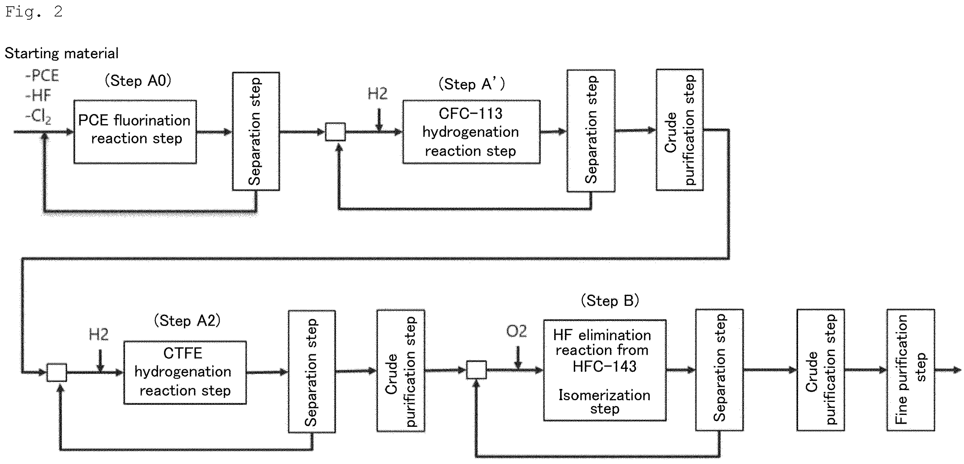

In another embodiment, the comprehensive process can comprise step A0, step A', step A2, and step B. This embodiment of the comprehensive process is referred to as "Comprehensive Process 2." Fig. 2 shows the concept of Comprehensive Process 2. In Fig. 2, PCE means perchloroethylene (tetrachloroethylene). This figure is a conceptual diagram of Comprehensive Process 2 and does not show all flows or processes.

In Comprehensive Process 2, step A' and step A2 are as follows.
Step A': collecting a fluoroethylene compound as an intermediate in the reduction reaction of step A.
Step A2: obtaining a fluoroethane compound by a vapor-phase hydrogenation reaction of the fluoroethylene compound.

In Comprehensive Process 2, step A0 is the same as or similar to step A0 of Comprehensive Process 1, and step B is the same as or similar to step B of Comprehensive Process 1.

In step A', the fluoroethylene compound is preferably at least one member selected from the group consisting of fluoroethylene (HFO-1141), 1,2-dichloro-1,2-difluoroethylene (CFO-1112), 1,1-difluoroethylene (HFO-1132a), 1,2-difluoroethylene (HFO-1132), chlorotrifluoroethylene (CTFE, CFO-1113), trifluoroethylene (HFO-1123), 2-chloro-1,1-difluoroethylene (HCFO-1122), 1-chloro-1,2-difluoroethylene (HCFO-1122a), and tetrafluoroethylene (FO-1114). Among these, the fluoroethylene more preferably comprises at least one member selected from the group consisting of chlorotrifluoroethylene (CTFE, CFO-1113) and tetrafluoroethylene (FO-1114); the fluoroethylene particularly preferably comprises chlorotrifluoroethylene (CTFE, CFO-1113).

In step A2, the method for producing a fluoroethane compound by a vapor-phase hydrogenation reaction of the fluoroethylene compound is not limited, and a wide range of known gas-phase hydrogenation reactions can be used. For example, it is possible to perform a hydrogenation reaction by allowing the fluoroethylene compound obtained in step A' and hydrogen gas to flow into a reactor filled with a catalyst to bring them into contact with the catalyst. A fluoroethane compound can thereby be obtained. The type of the fluoroethane compound is the same as or similar to the type of the fluoroethane compound produced by the reduction reaction of step A described above. When a gas-phase hydrogenation reaction of chlorotrifluoroethylene (CTFE, CFO-1113) is performed in step A', HFC-143 is obtained as the fluoroethane compound.

A fluoroolefin can be obtained by performing, in step B above, a dehydrohalogenation reaction of the fluoroethane compound obtained in step A2 above.

The various configurations (e.g., properties, structures, and functions) described in each of the above embodiments of the present disclosure may be combined in any way to specify the subject matter encompassed by the present disclosure. In other words, the present disclosure encompasses all the subjects comprising all combinations of the combinable configurations described in the present specification.

### Examples

The present invention is described in more detail below with reference to Examples. However, the present invention is not limited to the Examples.

### Example 1

One reactor with an inner diameter of 1.5 cm and a length of 50 cm was prepared, and 10 g of a catalyst in which 0.5% by mass of palladium (Pd) was supported relative to activated carbon was filled in the reactor. From the starting material supply port of this reactor, CFC-113 and hydrogen were supplied to the reactor and allowed to pass through the catalyst, and a reduction reaction was performed. In the reduction reaction, CFC-113 was supplied in gas form at a flow rate of 20 ml/min, and hydrogen gas was supplied so that the molar ratio of hydrogen:CFC-113 was 9:1 (i.e., the hydrogen/CFC-113 molar ratio was 9). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 3 g·sec/cc, and the temperature when CFC-113 and hydrogen passed through the catalyst (the reaction temperature, i.e., the temperature inside the reactor) was set to 300°C. As a result, HFC-143 was obtained. The conversion was 97% and the selectivity of HFC-143 was 77%. The selectivity of useful compounds, including intermediates that can be converted to HFC-143, was 85%.

### Example 2

HFC-143 was obtained in the same manner as in Example 1, except that the catalyst was changed to a catalyst in which 2% by mass of palladium (Pd) was supported relative to activated carbon. The conversion was 98%, and the selectivity of HFC-143 was 95%.

### Example 3

HFC-143 was obtained in the same manner as in Example 1, except that the catalyst was changed to a catalyst in which 3% by mass of palladium (Pd) was supported relative to activated carbon. The conversion was 99%, and the selectivity of HFC-143 was 98%.

### Examples 4 to 9

HFC-143 was obtained in the same manner as in Example 2, except that the reaction temperature and the hydrogen/CFC-113 molar ratio were set as shown in Table 1.

### Example 10

HFC-143 was obtained in the same manner as in Example 4, except that the catalyst was changed to a catalyst in which 2% by mass of Pt was supported relative to activated carbon.

### Example 11

HFC-143 was obtained in the same manner as in Example 4, except that the catalyst was changed to a catalyst in which 2% by mass of Ru was supported relative to activated carbon.

### Example 12

HFC-143 was obtained in the same manner as in Example 4, except that the catalyst was changed to a catalyst in which 2% by mass of Rh was supported relative to activated carbon.

### Comparative Example 1

HFC-143 was obtained in the same manner as in Example 1, except that the catalyst was changed to a catalyst in which 2% by mass of Pd was supported relative to aluminum oxide. The conversion here was 64%, and the selectivity was 40%. When the reaction was performed for 36 hours, the conversion was decreased to 40% and the selectivity was decreased to 32%.

Table 1 shows the reaction conditions of the reactions performed in the Examples (the reaction temperature and the hydrogen/CFC-113 molar ratio), as well as the conversion and selectivity after the reaction.

**Table 1**

| Example | Catalyst | Reaction temperature (°C) | Hydrogen/CFC-113 molar ratio | Conversion (%) | HFC-143 Selectivity (%) |
|---|---|---|---|---|---|
| 1 | Pd 0.5 wt%/C | 300 | 9 | 97 | 77 |
| 2 | Pd 2 wt%/C | 300 | 9 | 98 | 93 |
| 3 | Pd 3 wt%/C | 300 | 9 | 99 | 95 |
| 4 | Pd 2 wt%/C | 225 | 9 | 97 | 97 |
| 5 | Pd 2 wt%/C | 175 | 9 | 95 | 97 |
| 6 | Pd 2 wt%/C | 150 | 9 | 88 | 95 |
| 7 | Pd 2 wt%/C | 330 | 9 | 99 | 93 |
| 8 | Pd 2 wt%/C | 225 | 21 | 99 | 98 |
| 9 | Pd 2 wt%/C | 225 | 7 | 95 | 96 |
| 10 | Pt 2 wt%/C | 225 | 9 | 100 | 80 |
| 11 | Ru 2 wt%/C | 225 | 9 | 96 | 86 |
| 12 | Rh 2 wt%/C | 225 | 9 | 97 | 83 |

### Example 13

One reactor with an inner diameter of 0.97 cm and a length of 55 cm was prepared, and 2 g of a catalyst in which 2.0% by mass of palladium (Pd) was supported relative to activated carbon was filled in the reactor. From the starting material supply port of this reactor, CFC-113 with a water content of 41.8 ppm by mass and hydrogen were supplied to the reactor and allowed to pass through the catalyst, and a reduction reaction was performed. In the reduction reaction, CFC-113 was supplied in gas form at a flow rate of 4.7 ml/min, and hydrogen gas was supplied so that the molar ratio of hydrogen:CFC-113 was 12:1 (i.e., the hydrogen/CFC-113 molar ratio was 12). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 2 g·sec/cc, and the temperature when CFC-113 and hydrogen passed through the catalyst (the reaction temperature, i.e., the temperature inside the reactor) was set to 285°C. As a result, HFC-143 was obtained. The conversion was 100% and the selectivity of HFC-143 was 80%. The selectivity of useful compounds, including intermediates that can be converted to HFC-143, was 80%. The catalyst life under these conditions was about 46 hours, and when W/F was set to 12, the catalyst life was about 0.4 months.

### Example 14

One reactor with an inner diameter of 0.97 cm and a length of 55 cm was prepared, and 1.76 g of a catalyst in which 2.0% by mass of palladium (Pd) was supported relative to activated carbon was filled in the reactor. From the starting material supply port of this reactor, CFC-113 with a water content of 41.8 ppm by mass and hydrogen were supplied to the reactor and allowed to pass through the catalyst, and a reduction reaction was performed. In the reduction reaction, CFC-113 was supplied in gas form at a flow rate of 3.1 ml/min, and hydrogen gas was supplied so that the molar ratio of hydrogen:CFC-113 was 20:1 (i.e., the hydrogen/CFC-113 molar ratio was 20). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 1.65 g·sec/cc, and the temperature when CFC-113 and hydrogen passed through the catalyst (the reaction temperature, i.e., the temperature inside the reactor) was set to 175°C. As a result, HFC-143 was obtained. The conversion was 100% and the selectivity of HFC-143 was 56%. The selectivity of useful compounds, including intermediates that can be converted to HFC-143, was 88.5%. The catalyst life under these conditions was about 903 hours, and when W/F was set to 12, the catalyst life was about 9.1 months.

### Example 15

One reactor with an inner diameter of 0.97 cm and a length of 55 cm was prepared, and 1.76 g of a catalyst in which 2.0% by mass of palladium (Pd) was supported relative to activated carbon was filled in the reactor. From the starting material supply port of this reactor, CFC-113 with a water content of 41.8 ppm by mass and hydrogen were supplied to the reactor and allowed to pass through the catalyst, and a reduction reaction was performed. In the reduction reaction, CFC-113 was supplied in gas form at a flow rate of 1.4 ml/min, and hydrogen gas was supplied so that the molar ratio of hydrogen:CFC-113 was 25:1 (i.e., the hydrogen/CFC-113 molar ratio was 25). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 3 g·sec/cc, and the temperature when CFC-113 and hydrogen passed through the catalyst (the reaction temperature, i.e., the temperature inside the reactor) was set to 300°C. As a result, HFC-143 was obtained. The conversion was 100% and the selectivity of HFC-143 was 87%. The selectivity of useful compounds, including intermediates that can be converted to HFC-143, was 91.3%. The catalyst life under these conditions was about 952 hours, and when W/F was set to 12, the catalyst life was about 9.5 months.

### Example 16

One reactor with an inner diameter of 0.97 cm and a length of 55 cm was prepared, and 1.76 g of a catalyst in which 2.0% by mass of palladium (Pd) was supported relative to activated carbon was filled in the reactor. From the starting material supply port of this reactor, CFC-113 with a water content of 41.8 ppm by mass and hydrogen were supplied to the reactor and allowed to pass through the catalyst, and a reduction reaction was performed. In the reduction reaction, CFC-113 was supplied in gas form at a flow rate of 0.7 ml/min, and hydrogen gas was supplied so that the molar ratio of hydrogen:CFC-113 was 51:1 (i.e., the hydrogen/CFC-113 molar ratio was 51). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 3 g·sec/cc, and the temperature when CFC-113 and hydrogen passed through the catalyst (the reaction temperature, i.e., the temperature inside the reactor) was set to 300°C. As a result, HFC-143 was obtained. The conversion was 100% and the selectivity of HFC-143 was 83%. The selectivity of useful compounds, including intermediates that can be converted to HFC-143, was 86.1%. The catalyst life under these conditions was about 1120 hours, and when W/F was set to 12, the catalyst life was about 11.2 months.

### Example 17

One reactor with an inner diameter of 0.97 cm and a length of 55 cm was prepared, and 1.42 g of a catalyst in which 2.0% by mass of palladium (Pd) was supported relative to activated carbon was filled in the reactor. From the starting material supply port of this reactor, CFC-113 with a water content of 510 ppm by mass and hydrogen were supplied to the reactor and allowed to pass through the catalyst, and a reduction reaction was performed. In the reduction reaction, CFC-113 was supplied in gas form at a flow rate of 4.1 ml/min, and hydrogen gas was supplied so that the molar ratio of hydrogen:CFC-113 was 10:1 (i.e., the hydrogen/CFC-113 molar ratio was 10). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 1.72 g·sec/cc, and the temperature when CFC-113 and hydrogen passed through the catalyst (the reaction temperature, i.e., the temperature inside the reactor) was set to 175°C. As a result, HFC-143 was obtained. The conversion was 85% and the selectivity of HFC-143 was 50%. The selectivity of useful compounds, including intermediates that can be converted to HFC-143, was 86%. The catalyst life under these conditions was about 540 hours, and when W/F was set to 12, the catalyst life was about 5.2 months.

### Example 18

One reactor with an inner diameter of 0.97 cm and a length of 55 cm was prepared, and 1.42 g of a catalyst in which 2.0% by mass of palladium (Pd) was supported relative to activated carbon was filled in the reactor. From the starting material supply port of this reactor, CFC-113 with a water content of 173 ppm by mass and hydrogen were supplied to the reactor and allowed to pass through the catalyst, and a reduction reaction was performed. In the reduction reaction, CFC-113 was supplied in gas form at a flow rate of 4.1 ml/min, and hydrogen gas was supplied so that the molar ratio of hydrogen:CFC-113 was 10:1 (i.e., the hydrogen/CFC-113 molar ratio was 10). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 1.72 g·sec/cc, and the temperature when CFC-113 and hydrogen passed through the catalyst (the reaction temperature, i.e., the temperature inside the reactor) was set to 175°C. As a result, HFC-143 was obtained. The conversion was 99.9% and the selectivity of HFC-143 was 57.5%. The selectivity of useful compounds, including intermediates that can be converted to HFC-143, was 89.1%. The catalyst life under these conditions was about 455 hours, and when W/F was set to 12, the catalyst life was about 4.4 months.

### Example 19

One reactor with an inner diameter of 0.97 cm and a length of 55 cm was prepared, and 1.46 g of a catalyst in which 2.0% by mass of palladium (Pd) was supported relative to activated carbon was filled in the reactor. The pressure inside the reactor was adjusted to 0.20 MPaG. From the starting material supply port of this reactor, CFC-113 with a water content of 173 ppm by mass and hydrogen were supplied to the reactor and allowed to pass through the catalyst, and a reduction reaction was performed. In the reduction reaction, CFC-113 was supplied in gas form at a flow rate of 4.7 ml/min, and hydrogen gas was supplied so that the molar ratio of hydrogen:CFC-113 was 10:1 (i.e., the hydrogen/CFC-113 molar ratio was 10). In the reduction reaction, the contact time represented by W/F, i.e., the ratio of the catalyst amount in the reactor W (g) to the total flow rate of CFC-113 and hydrogen gas introduced into the reactor F (the flow rate at 25°C and 0.1013 MPa), was set to 1.70 g·sec/cc, and the temperature when CFC-113 and hydrogen passed through the catalyst (the reaction temperature, i.e., the temperature inside the reactor) was set to 175°C. As a result, HFC-143 was obtained. The conversion was 99.9% and the selectivity of HFC-143 was 50.9%. The selectivity of useful compounds, including intermediates that can be converted to HFC-143, was 89.8%. The catalyst life under these conditions was about 569 hours, and when W/F was set to 12, the catalyst life was about 5.6 months.

## Claims

1. A method for producing a fluoroethane compound, comprising
step A of obtaining a product comprising a fluoroethane compound by performing a reduction reaction of a chlorofluoroethane compound in the presence of a catalyst and hydrogen,
wherein the catalyst is a catalyst in which at least one metal selected from the group consisting of Ni, Pd, Pt, Ru, and Rh is supported on activated carbon.

2. The production method according to claim 1,
wherein the fluoroethane compound comprises 1,1,2-trifluoroethane, and the chlorofluoroethane compound comprises 1,1,2-trichloro-1,2,2-trifluoroethane.

3. The production method according to claim 1,
wherein the fluoroethane compound comprises 1,1,2-trifluoroethane, and the chlorofluoroethane compound comprises 1-chloro-1,1,2-trifluoroethane.

4. The production method according to claim 1,
wherein the fluoroethane compound comprises 1,1-difluoroethane, and the chlorofluoroethane compound comprises 1-chloro-1,1,2-trifluoroethane.

5. The production method according to claim 2, wherein the product comprises at least one compound selected from the group consisting of chlorotrifluoroethylene and trifluoroethylene as a by-product.

6. The production method according to claim 2,
comprising
obtaining the 1,1,2-trichloro-1,2,2-trifluoroethane by a liquid-phase fluorination reaction of tetrachloroethylene.

7. A method for producing a fluoroolefin, comprising
obtaining a fluoroethane compound by the production method of any one of claims 1 to 6, and
obtaining the fluoroolefin by a dehydrofluorination reaction of the fluoroethane compound obtained in the above step.
